(19) Europäisches Patentamt
European Patent Office
Office européen des brevets

(11) EP 4 216 989 B1

(12) EUROPEAN PATENT SPECIFICATION

(45) Date of publication and mention
of the grant of the patent:
29.04.2026 Bulletin 2026/18

(21) Application number: 21785999.0

(22) Date of filing: 21.09.2021

(51) International Patent Classification (IPC):
A61K 38/40 (2006.01)   A61K 33/00 (2006.01)
A61K 47/12 (2006.01)   A61P 31/04 (2006.01)
A61P 31/12 (2006.01)   C07K 14/79 (2006.01)
C11D 3/10 (2006.01)   C11D 3/20 (2006.01)
A61K 47/02 (2006.01)

(52) Cooperative Patent Classification (CPC):
(C-Sets available)
A61K 38/40; A61K 9/0034; A61K 9/0043;
A61K 9/08; A61K 31/194; A61K 33/00;
A61K 47/12; A61P 31/04; A61P 31/12; C07K 14/79
(Cont.)

(86) International application number:
PCT/IB2021/058581

(87) International publication number:
WO 2022/064357 (31.03.2022 Gazette 2022/13)

(54) **FORMULATIONS FOR TOPIC MUCOSAL AND/OR ORAL SYSTEMIC ADMINISTRATIONS CONTAINING A MIXTURE BASED ON LACTOFERRIN, MORE EFFECTIVE IN IRON UPTAKE, FOR THE PREVENTION AND TREATMENT OF VARIOUS PATHOLOGIES**

FORMULIERUNGEN FÜR DIE MUKOSALE UND/ODER ORALE SYSTEMISCHE VERABREICHUNG, DIE EIN GEMISCH AUF DER BASIS VON LACTOFERRIN ENTHALTEN, DAS BEI DER EISENAUFNAHME WIRKSAMER IST, ZUR VORBEUGUNG UND BEHANDLUNG VON VERSCHIEDENEN KRANKHEITEN

FORMULATIONS POUR ADMINISTRATIONS SYSTÉMIQUES TOPIQUES MUQUEUSES ET/OU ORALES CONTENANT UN MÉLANGE À BASE DE LACTOFERRINE, PLUS EFFICACE DANS L'ABSORPTION DU FER, POUR LA PRÉVENTION ET LE TRAITEMENT DE DIVERSES PATHOLOGIES

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 23.09.2020 IT 202000022420

(43) Date of publication of application:
02.08.2023 Bulletin 2023/31

(73) Proprietor: Microbo S.r.l.
00153 Roma (RM) (IT)

(72) Inventors:
• VALENTI, Piera
00153 Roma RM (IT)
• PAESANO, Rosalba
00153 Roma RM (IT)

(74) Representative: Perani & Partners S.p.A.
Corso Europa, 15
20122 Milano (IT)

(56) References cited:
EP-B1- 0 454 084      WO-A1-2007/065482
WO-A1-2012/084459      WO-A1-2014/097123
WO-A1-2020/194225

- MAZURIER J ET AL: "Comparative study of the iron-binding properties of human transferrins II. Electron paramagnetic resonance of mixed metal complexes of human lactotransferrin", BIOCHIMICA ET BIOPHYSICA ACTA. PROTEIN STRUCTURE AND MOLECULAR ENZYMOLOGY, ELSEVIER, AMSTERDAM; NL, vol. 745, no. 1, 30 May 1983 (1983-05-30), pages 44 - 49, XP023475768, ISSN: 0167-4838, [retrieved on 19830530], DOI: 10.1016/0167-4838(83)90168-1
- ADLEROVA L ET AL: "Lactoferrin: a review", vol. 53, no. 9, 15 September 2008 (2008-09-15), pages 457 - 468, XP002788307, ISSN: 0375-8427, Retrieved from the Internet <URL:https://www.vri.cz/docs/vetmed/53-9-457.pdf> DOI: 10.17221/1978-VETMED
- STRATE VAN DER B W A ET AL: "Antiviral activities of lactoferrin", ANTIVIRAL RESEARCH, ELSEVIER BV, NL, vol. 52, no. 3, 1 December 2001 (2001-12-01), pages 225 - 239, XP002325824, ISSN: 0166-3542, DOI: 10.1016/S0166-3542(01)00195-4
- PIERA VALENTI ET AL: "Role of Lactobacilli and Lactoferrin in the Mucosal Cervicovaginal Defense", FRONTIERS IN IMMUNOLOGY, vol. 9, 1 March 2018 (2018-03-01), XP055744173, DOI: 10.3389/fimmu.2018.00376
- LEPANTO MARIA STEFANIA ET AL: "Lactoferrin in Aseptic and Septic Inflammation", MOLECULES, vol. 24, no. 7, 3 April 2019 (2019-04-03), DE, pages 1323, XP055808182, ISSN: 1433-1373, DOI: 10.3390/molecules24071323
- NONNECKE B J ET AL: "Inhibition of mastric bacteria by bovine milk apo-lactoferrin evaluated by in vitro microassay of bacterial growth", INDIAN JOURNAL OF DAIRY SCIENCE,, vol. 67, no. 3, 1 January 1984 (1984-01-01), pages 606 - 613, XP003019976
- JIANSHE LANG ET AL: "Inhibition of SARS Pseudovirus Cell Entry by Lactoferrin Binding to Heparan Sulfate Proteoglycans", PLOS ONE, vol. 6, no. 8, 1 August 2011 (2011-08-01), pages e23710, XP055765756, DOI: 10.1371/journal.pone.0023710
- MIRABELLI CARMEN ET AL: "Morphological Cell Profiling of SARS-CoV-2 Infection Identifies Drug Repurposing Candidates for COVID-19", BIORXIV, 10 June 2020 (2020-06-10), pages 1 - 36, XP055872688, Retrieved from the Internet <URL:https://www.biorxiv.org/content/10.1101/2020.05.27.117184v2.full.pdf> [retrieved on 20211214], DOI: 10.1101/2020.05.27.117184

(52) Cooperative Patent Classification (CPC): (Cont.)

C-Sets
A61K 31/194, A61K 2300/00;
A61K 33/00, A61K 2300/00;
A61K 38/40, A61K 2300/00

**Description**

**FIELD OF THE INVENTION**

The present invention relates to an innovative composition for significantly increasing the efficacy of natural lactoferrin of any origin or recombinant lactoferrin, in chelating iron, significantly increasing the activity thereof in decreasing the excess of available free iron, index of a pathological disorder which significantly favours bacterial, fungal and viral infections and the onset of a pathological state associated with iron homoeostasis and inflammation disorders such as in Alzheimer's, IBD or preterm childbirth.

**BACKGROUND ART**

While protected from commensal microbiota, human mucous membranes are exposed to attack by numerous pathogenic microorganisms. For this reason, in addition to the commensal microbiota which prevents the colonisation of pathogens, the mucous membranes are wetted with secretions which, consisting of innate immunity proteins and peptides, ensure an effective and complete protection from infections by pathogenic bacteria and viruses. Among the innate immunity proteins, lactoferrin is of particular importance, being the only one with a strong antibacterial and antiviral activity as well as a remarkable multi-functionality (Rosa et al 2017). Lactoferrin, a highly cationic bilobed glycoprotein (N-lobe and C-lobe) belonging to the transferrin family with a PM of about 80 kDa, can chelate two ferric ions per molecule. This primary function allows it to subtract iron from the microorganisms which need it for multiplication. Like all iron-binding proteins, lactoferrin sequesters iron through four canonical amino acids which form the chelating site. Such a site is present in each lobe and is formed in lobe N by Asp58, Tyr93, Tyr193 and His254 while in lobe C by Asp396, Tyr434, Tyr527 and His596. The stability of said binding varies according to the temperature, pH and integrity of the glycoprotein (Rosa et al 2018) which can be digested by proteolytic enzymes in fragments lacking the amino acid sequences fundamental for sequestering the ferric ion. Iron, on the other hand, is important for almost all living cells as microorganisms, in order to acquire it, have developed multiple transport systems ranging from the synthesis of siderophores, small PM molecules of about 600 kDa capable of chelating one ferric ion per molecule (Petrik et al 2017), to that of enzymes with iron-reductase activity which reduce ferric ions into easily acquired ferrous ions and to iron uptake directly by the lactoferrin (Beddek and Schryvers 2010). The microorganisms thus engage a real battle of chelating agents with the lactoferrin, whose result depends on the infections and diseases of the host. Obviously, the outcome of this battle depends on the different affinity for the ferric ion possessed by the various microbial acquisition systems with respect to that of the lactoferrin. Although lactoferrin is the glycoprotein with the highest affinity for the ferric ion (Kd of about $10^{-20}$ M) among the proteins belonging to the family of transferrins, under particular conditions the iron may or may not be released. Particularly interesting is the fact that, unlike commensal microorganisms, pathogenic bacteria can, like viruses, replicate within host cells. It follows that an excess of intracellular iron favours not only bacterial but also viral replication, thus worsening the severity of infections. Thus, the ability of lactoferrin to sequester iron entails an inhibitory action against bacterial and viral multiplication, and therefore, by right, it is classified as a natural protein with antimicrobial and antiviral activity (Valenti and Antonini 2005). With bacteria multiplication inhibited, they do not reach a cell density such as to activate the quorum sensing sequences which lead to the formation and development of biofilm (set of bacteria and the exopolysaccharide synthesised thereby), a particular microbial lifestyle very difficult to eradicate with the antimicrobial drugs known so far. If the replication of the viruses is inhibited inside the host cells, the viral infection is inhibited. Furthermore, lactoferrin also inhibits bacterial and viral adhesion to host cells and, consequently, bacterial and viral internalisation into host cells. These inhibitions are due to a binding of lactoferrin to the negatively charged surface components of bacteria and viruses and/or the binding of lactoferrin to the glycosaminoglycans of the cell, compounds of the extracellular matrix used by bacteria and viruses to adhere to and then enter the cell (Rosa et al 2017; Mancinelli et al 2020; Sample et al 2020). Furthermore, if the number of intracellular bacteria or pathogenic viruses decreases, the production of pro-inflammatory cytokines by the infected epithelial cells and therefore the inflammatory process also decreases, although it cannot be excluded that the anti-inflammatory activity also depends on the lactoferrin itself which is able to enter the nucleus where it binds to sequences encoding pro-inflammatory cytokines (Puddu et al 2011; Paesano et al 2012; Rosa et al 2017), mainly including interleukin-6 (IL-6). A pathological increase in IL-6 induces disorders in iron homoeostasis and inflammation, through an inhibition of ferroportin, the only protein capable of exporting iron from cells into circulation. Consequently, in the absence of ferroportin, the harmful accumulation of intracellular iron is observed. In the presence of lactoferrin, thanks to its ability to chelate iron it decreases its accumulation as well as its anti-inflammatory action, ferroportin is synthesised again, thus reactivating the export of iron which decreases the intracellular accumulation of such an element.

In summary, in all diseases associated with an inflammatory process, a disorder of iron homoeostasis occurs which consists of an iron overload in tissues and secretions which favours bacterial and viral infections and induces cellular damage due to the formation of reactive iron-dependent oxygen species. Consequently, lactoferrin can be used in the prevention and treatment of all diseases in which an accumulation of intra and extracellular iron is found and, therefore, not only of bacterial and viral infections but also in Alzheimer's, where said glycoprotein decreases the cerebral accumulation of iron and the production of pro-inflammatory cytokines, in IBD where it contributes to the restoration of the integrity of the intestinal barrier and the decrease of the related inflammatory processes and also in preterm childbirth, where it performs an antibacterial, antifungal, antiviral and anti-inflammatory action.

WO 2007065482A1 discloses the use of at least one transferrin, preferably lactoferrin. To prepare a medicament for preventing and/or treating with great effectiveness destructive inflammation affecting mucous membranes optionally associated with states of hypoferremia and/or anemia and/or acute and chronic infections.

EP 0454084B1 describes a method of manufacturing either aqueous solution of lactoferrin, iron preparation and sodium bicarbonate, or aqueous solution of iron lactoferrin; and adjusting its electric conductivity (OMEGA mS/cm) and its hydrogen ion concentration (pH) so that it meets the conditions provided by the following formulae:

$$\log \Omega \leqq (2.96/pH) + 0.64 \ pH < 5$$

$$\log \Omega \leqq (29.37/pH) - 4.62 \ 5 \leqq pH \leqq 7.9$$

$$\log \Omega \leqq - 0.917 \ pH > 7.9$$

pasteurizing by heat; and manufacturing iron-fortified beverage using above materials; and having high bioavailability of iron and maintaining physiological functions of lactoferrin.

XP 002788307 is a review discussing the biological properties of lactoferrin such as it role in iron metabolism, cell proliferation and differentiation, antibacterial, antiviral and antiparasitic activity.

XP 002325824 is an overview of the antiviral activity in preventing entry of the virus in the host cell by blocking cellular receptors or by direct binding to the virus particles. XP 0055744173 disclose the role of lactobacilli and lactoferrin in the mucosal and cervico vaginal defence especially in counteracting recurrent and/or antibiotic-resistant bacterial infections.

XP 0055808182 discloses that high levels of IL-6 are involved in iron homeostasis disorders lactoferrin is merging as a potent regulator of iron and inflammatory homeostasis. In particular the role of lactoferrin as against antiseptic and septic inflammation has been reviewed. In aseptic inflammation as anaemia of inflammation, preterm delivery, Alzheimer's disease and type 2 diabetes lactoferrin reduces local and/or systemic inflammation. In septic inflammation occurring in Chlamidia trachomatis infections, cystic fibrosis and inflammatory, lactoferrin besides, besides the anti-inflammatory activity, exerts a significant activity against bacterial adhesion , invasion and colonization.

WO 2014097123A1 discloses a composition containing lactoferrin and its use in preventing preterm delivery.

WO 2012084459A1 discloses for preventing and treating preterm delivery.

XP 003019976 discloses that bicarbonate supplementation of the growth system containing apolactoferrin (1 mg/ml) increased the inhibition of three coliform strains by apolactoferrin. Addition of increasing concentration of citrate (2.0 mg/ml) to an assay system containing apolactoferrin (5 mg/ml) resulted in a concomitant reduction of growth inhibition of three coliform strains. These data indicate a potential relationship between the molar ratio of citrate to lactoferrin of the lacteal secretion and its capacity to inhibit coliform strain associated with mastitits.

WO 2020194225A1 relates to a mixture containing lactoferrin, sodium citrate and sodium bicarbonate and whose molar ratio intended as moles of lactoferrin/ (moles of sodium citrate+ moles of sodium bicarbonate) is between $10^{-4}$ and $10^{-3}$ for use in the treatment of infections colonising the epithelia in the oral mucous membranes for the purpose of: decreasing the number of pathogenic microorganisms, including those associated with tooth pigmentation such as black stains, inhibiting the formation of biofilm, inhibiting the microbial ability to adhere and invade, helping injury repair, inhibiting or decreasing the inflammatory process caused by infections optionally associated with iron deficiency, anaemia, inflammation anaemia or diabetes. The mixture is administered only topically in the form of an oral topical formulation in the absence of iron homeostasis disorders or the oral topical formulation is administered in combination with a systemic oral formulation including lactoferrin, sodium citrate and sodium bicarbonate in the same molar ratio range as above, when these infections and the associated inflammatory process in the oral mucous membranes are associated with iron deficiency, anaemia or inflammation anaemia, or when the inflammatory process is associated with diabetes.

XP 005576575 discloses that lactoferrin inhibits SARS pseudo virus infection in a dose dependant manner.

XP 0055872688 discloses that lactoferrin id an effective inhibitor of SARS-CoV-2 infection with an IC50 of 308nM and that it potentiates the efficacy of remdesivir and hydroxychloroquine.

XP0023475768 discloses that human lactoferrin is able to bind two vanadyl (IV) ions specific to specific metal -binding site, although the EPR signal appear as one suggesting that the environments of the binding site of human lactoferrin are similar. The binding activity is promoted to pH 4 using carbonate or bicarbonate as synergistic action, but is destroyed below pH6 for other transferrins.

## SUMMARY OF THE INVENTION

The Applicant has now found that it is possible to enhance the iron-chelating activity of lactoferrin by adding sodium citrate and sodium bicarbonate in molar ratio understood as: moles lactoferrin/ (moles sodium citrate + moles sodium bicarbonate) between $10^{-4}$ and $10^{-3}$..

In fact, this combination administered for topical and/or oral use is capable of performing a more powerful antimicrobial and antiviral activity, subtracting iron more effectively and chelating it more stably than lactoferrin alone or in the absence of sodium citrate and sodium bicarbonate or added with sodium citrate alone or sodium bicarbonate alone.

The Applicant has further found that lactoferrin can be effectively used when administered only orally at concentrations > 200mg up to 500mg, in this case using appropriate ranges of concentrations of the above salts, as we will report in the continuation of the present disclosure.

In fact, as claimed in claim 1 the object of the present invention is a topical nasal, vaginal or anal formulation A) comprising as active ingredient a mixture containing lactoferrinsodium citrate and sodium bicarbonate in the molar ratio range:

$$(moli\ di\ lattoferrina)/(moli\ di\ sodio\ citrato\ +\ moli\ di\ sodio\ bicarbonato)$$

between $10^{-4}$ and $10^{-3}$ for use in the treatment or prevention of bacterial and viral infections which colonise the epithelia at the nasal, vaginal, cervicovaginal, anal and intestinal mucosal levels optionally associated with iron deficiency, anaemia or inflammatory anaemia,

said topical formulation A) being administered only nasally, intravaginally to the nasal, vaginal and cervicovaginal mucous membranes or topically intra-anally to the anal and intestinal mucous membranes, in the absence of iron homoeostasis disorders, or

said topical formulation A) being administered nasally, vaginally or anally in combination with a systemic oral formulation B), containing said mixture wherein the lactoferrin, sodium citrate and sodium bicarbonate are present in the same molar ratio range as above, when said mucosal infections and the inflammatory process thereof manifest with iron deficiency, anaemia or inflammatory anaemia, or

the aforementioned systemic oral formulation B) is administered in combination with said nasal topical formulation A), when cerebral iron overload and the inflammatory processes thereof are associated with Alzheimer's.

In fact, the applicant has found that both formulations A) and B) containing the above mixture of lactoferrin sodium citrate and sodium bicarbonate in the aforementioned moral ratio ranges are able to chelate iron with greater effectiveness than lactoferrin alone, or added with sodium citrate alone, or with sodium bicarbonate alone, and to exercise greater anti-inflammatory activity to be used in the treatment and prevention of infections by bacteria and viruses, which colonise the nasal, pulmonary, intestinal, vaginal, cervicovaginal and anal epithelia so as to:

- decrease the number of pathogenic bacteria and viruses, including SARS-CoV-2.

- inhibit biofilm formation,

- decrease the ability of bacteria and viruses to adhere and penetrate inside the host cell.

Furthermore, through the decrease in iron overload and inflammation lactoferrin is also capable of:

- inhibiting or decreasing the accumulation of iron and the inflammatory process associated with Alzheimer's,

- preventing and treating IBD,

- repairing lesions by restoring the integrity of the intestinal mucous membranes,

- inhibiting infections and decreasing the inflammation which causes preterm childbirth The formulation A) according to the invention preferably contains lactoferrin in amounts preferably between 10 and 200 mg, more preferably at concentrations comprised between 50 and 100 mg is administered exclusively topically intranasally, intravaginally, cervicovaginally or intra-anally in the form of a topical formulation A) nasally, vaginally or anally in the absence of iron homoeostasis disorders; it is instead administered in combination with a systemic oral formulation B) containing the lactoferrin, sodium citrate and sodium bicarbonate in the same molar ratio range as above, when the aforementioned diseases occur with iron deficiency, anaemia or inflammatory anaemia or the aforementioned systemic oral formulation B) is administered in combination with:

- said topical formulation A) nasally when cerebral iron overload and related inflammatory processes are associated with Alzheimer's,

- said topical formulation A) anally when the intestinal infection and inflammatory intestinal process is associated with the inflammatory process is related to Inflammatory Bowel Disease (IBD),

- said topical formulation A) vaginally when the vaginal infectious and inflammatory process is associated with preterm childbirth.

The oral formulation B) optionally administered with the topical formulation A) for the use according to the present invention preferably contains lactoferrin in amounts ranging from 100 to 200mg to be administered from two to several times.

A further subject of the invention is the second pharmaceutical use claimed in claim 13.

In fact, the Applicant has found that the oral formulation ) containing lactoferrin > 200 up to 500 mg, preferably between 250 and 500 mg, and a total sodium citrate and sodium bicarbonate content comprised between 140 and 240 mg, preferably between 170 and 200 mg in which each of said salts is present in an amount between 15 and 190 mg, preferably between 20 and 160 mg, it is administered exclusively orally, preferably twice a day and has been particularly effective in the prevention and treatment of viral infections from DNA, RNA, enveloped or naked, in particular from SARS-CoV-2 or other diseases in which an intracellular and extracellular iron overload and an aggressive inflammatory response are observed.

## DESCRIPTION OF THE FIGURES

Figure 1 depicts the ferric ion uptake site of lactoferrin containing the citrate in lobe N.

Figure 2 depicts the ferric ion uptake site of lactoferrin containing the bicarbonate ion in lobe N. Key to the figures:

His=histidine; Tyr=tyrosine; Asp=asparagine; Arg=arginine. The same identical bond can be observed in lobe C.

## DETAILED DESCRIPTION OF THE INVENTION

For the purposes of the present invention lactoferrin means lactoferrin of any origin (human, bovine, equine, murine, caprine, camel, buffalo and donkey) and recombinant lactoferrins.

Without wishing to be bound to any scientific theory, the enhancement of the iron-chelating activity seems to occur according to the following pattern of reactions: [1]

*2 sodium citrate + $Fe^{3+}$ → ferric dicitrate (monocitrate-$Fe^{3+}$-sodium monocitrate) ferric dicitrate+ sodium bicarbonate → bicarbonate-$Fe^{3+}$ + 2 sodium monocitrate bicarbonate$Fe^{3+}$ + lactoferrin → lactoferrin-$Fe^{3+}$- sodium bicarbonate* **[1]**

This reaction, which highlights the ability of sodium bicarbonate to displace citrate in ferric ion uptake, does not however exclude the possibility that ferric monocitrate may form a complex with lactoferrin and then be displaced from the bicarbonate which stabilises the lactoferrin-$Fe^{3+}$ bond according to the following pattern of reactions **[2]**:

*2 sodium citrate + $Fe^{3+}$ → ferric dicitrate (monocitrate-$Fe^{3+}$-sodium monocitrate) ferric monocitrate + lactoferrin → lactoferrin-$Fe^{3+}$-sodium citrate lactoferrin-$Fe^{3+}$-citrate + sodium bicarbonate → lactoferrin- $Fe^{3+}$- bicarbonate + sodium monocitrate* **[2]**

In other words, the sodium citrate is used to solubilise and chelate the ferric ion through the formation of the ferric dicitrate, which in turn exchanges the iron with the bicarbonate according to the following reaction: **[3]**

*ferric dicitrate + sodium bicarbonate → bicarbonate$Fe^{3+}$ + 2 sodium monocitrate* **[3]**

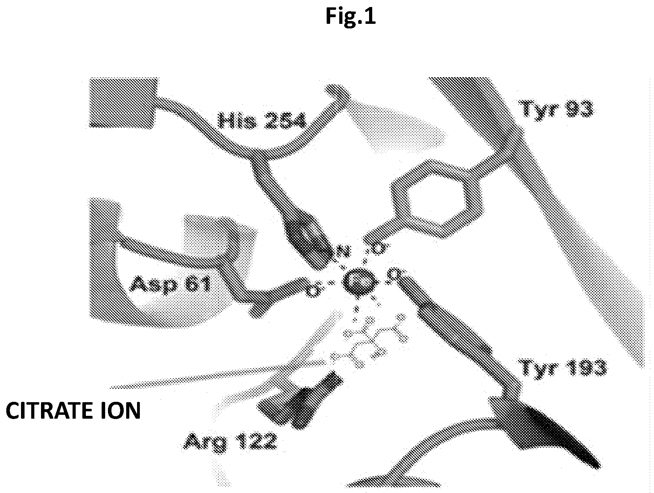

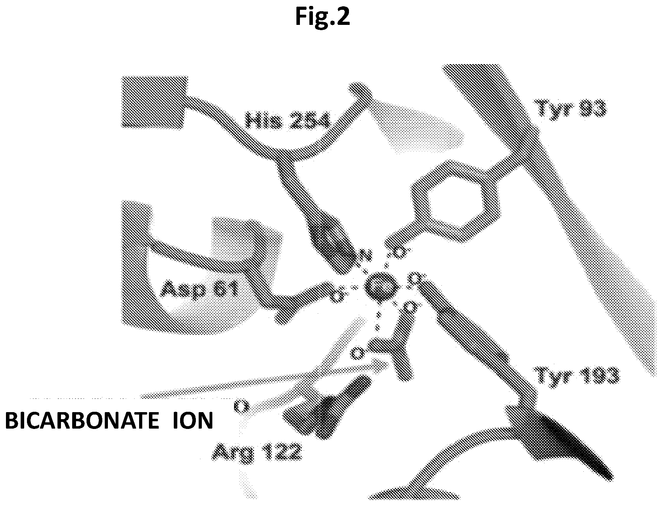

or forms a complex with lactoferrin (lactoferrin-$Fe^{3+}$-citrate) from which it is then displaced by the bicarbonate (lactoferrin-$Fe^{3+}$-bicarbonate) returning to sodium monocitrate, which in turn reactivates the uptake cycle of the available ferric ions. Ferric citrate and ferric bicarbonate are ideal compounds for transporting the ferric ion to one of the two lactoferrin uptake

6

sites, according to the pattern shown in Figures 1 and 2, respectively.

Highly conserved in various species, lactoferrin is a glycoprotein consisting of 685 to 691 amino acids depending on the species, with an isoelectric point of approximately 9 and a molecular weight of approximately 80 kDa with 3 or 5 glycosylation sites (human or bovine, respectively). The binding affinity with iron is equal to Kd of about $10^{-20}$ M which increases significantly in the mixture described above and is greater than the transferrins which are responsible for the transport of iron and therefore release it more easily (Rosa et al 2017). Native lactoferrin is produced in large amounts by extraction from bovine milk, whereas recombinant human or bovine lactoferrin is produced by recombinant DNA. Preferably bovine lactoferrin is used.

The term "mixture" in the formulations for the uses according to the invention means a composition having as active ingredients lactoferrin, preferably of bovine origin, associated with sodium citrate and sodium bicarbonate in which the molar ratio:

(moli di lattoferrina)/(moli di sodio citrato + moli di sodio bicarbonato) is between $10^{-4}$ and $10^{-3}$.

Therefore for the use claimed in claim 1, the preferred concentration of lactoferrin ranges from 10 up to 200 mg in the topical formulation A) optionally associated with the oral formulation B) the latter containing lactoferrin in amounts comprised preferably between 100 and 200 mg.

if For the use claimed in claim 13 the lactoferrin is used at a concentration of > 200 up to 500 mg, preferably between 250 and 500 mg with a total content of sodium citrate and sodium bicarbonate comprised between 140 and 240 mg, preferably between 170 and 200 mg, i.e., with an amount of each of said salts between 15 and 190 mg, preferably between 20 and 160 mg. The mixture with the lactoferrin at the concentration up to 200 mg can be administered topically (intranasally, intravaginally or intra-anally) in a liquid, semi-solid, solid or granular form containing in addition to the lactoferrin, sodium citrate and sodium bicarbonate, excipients and/or diluents for conventional use and suitable for the specific type of administration.

The mixture thus composed of lactoferrin, sodium citrate and sodium bicarbonate chelates the ferric ion more effectively, as shown in Table 1 below, and is applied in the prevention and treatment of bacterial and viral infections, as well as in the reduction of excess intra- and extra-cellular iron, inflammation and in the protection of lesions of the nasal, vaginal, anal and intestinal mucous membranes.

Preferably in the mixture contained in the formulations for the uses claimed the molar ratio lactoferrin moles/(-moles sodium bicarbonate+sodium citrate) is between 3 and 7 $\times 10^{-4}$.

The mixture is administered only intranasally or intravaginally or intra-anally, when the infection with bacteria, fungi or pathogenic viruses is associated with inflammation of the nasal or vaginal mucosa or cervicovaginal or intestinal mucosa but is not associated with iron deficiency or iron-deficiency anaemia or inflammatory anaemia. In addition, the intranasal mixture is also administered to decrease cerebral iron overload such as in Alzheimer's. This topical application is combined with the administration of a systemic oral formulation, when the infection is related to bacteria, fungi or pathogenic viruses or cerebral iron overload is associated with inflammation and also with iron deficiency, iron-deficiency anaemia or inflammatory anaemia.

The mixture is administered only topically intra-anally, as an anal formulation when the microbial infection is associated with inflammation of the anal and intestinal mucous membranes and in particular the infection is associated with bacteria, fungi or pathogenic viruses, but is not associated with iron deficiency or iron-deficiency anaemia or inflammatory anaemia. This topical application is combined with systemic oral administration, when the infection related to bacteria, fungi or pathogenic viruses is associated with inflammation and also with iron deficiency, iron-deficiency anaemia or inflammatory anaemia.

The mixture is administered only topically intravaginally or cervicovaginally in the form of a topical formulation A) intravaginally or cervicovaginally when the microbial infection is associated with inflammation of the vagina and/or cervix and in particular the infection is associated with bacteria, fungi or pathogenic viruses, but is not associated with iron deficiency or iron-deficiency anaemia or inflammatory anaemia. This topical application is combined with systemic oral administration when the infection related to bacteria, fungi or pathogenic viruses is associated with inflammation and also with iron deficiency, iron-deficiency anaemia or inflammatory anaemia.

The mixture is administered exclusively orally when the lactoferrin is > 200 up to 500 mg with a total sodium citrate and sodium bicarbonate content comprised between 140 and 240 mg with an amount of each of said salts comprised between 15 and 190 mg, preferably between 20 and 160 mg and the infection is associated with SARS-CoV-2 or its variants or pathologies in which there is iron overload and a severe inflammatory state. In fact, the mixture thus composed of lactoferrin, sodium citrate and sodium bicarbonate chelates the ferric ion more effectively, as demonstrated in Table 2 below, and is applied in the prevention and treatment of bacterial and viral infections, as well as in reducing intra and extracellular iron excess and inflammation.

For all the above reasons, the combined use of the topical formulation (intranasally or intravaginally or intra-anally) of the mixture with the systemic oral formulation, if the lactoferrin is administered at a concentration comprised between 10 and 200 mg, represents the best treatment not only of bacterial and viral infections of the mucous membranes, but also of nasal, cerebral, vaginal or intestinal inflammations including Alzheimer's, IBD and preterm childbirth as well as of iron-deficiency diseases, iron-deficiency anaemia and inflammatory anaemia.

More generally, when the inflammation of the mucous membranes derives from a pathological accumulation of available iron, from the production of reactive oxygen species and from a high bacterial and viral replication, a consequent activation of inflammatory processes optionally associated with iron deficiency or iron-deficiency anaemia or inflammatory anaemia is observed and systemic topical and oral treatment is preferred.

The mixture containing lactoferrin of any natural or recombinant origin, sodium citrate and sodium bicarbonate in the above molar ratio ranges, or in the amounts cited administered for topical (intranasal or intravaginal or intraanal) and/or systemic oral use, in fact simultaneously performs:

i) antimicrobial activity against pathogenic bacteria as well as iron-dependent antiviral activity and protection against infection,

ii) iron-dependent anti-biofilm activity,

iii) activity which inhibits the production of reactive iron-dependent oxygen species,

iv) activity inhibiting inflammatory processes induced by reactive iron-dependent oxygen species,

v) antiadhesive activity against bacteria or viruses dependent on interaction with surface components negatively loaded with bacteria and viruses and/or with cellular surface anionic components,

vi) activity inhibiting the entry of bacteria or viruses dependent on the interaction with surface components negatively charged with bacteria and viruses and/or with cellular surface anionic components,

vii) anti-inflammatory activity against the expression and synthesis of pro-inflammatory cytokines such as IL-1$\beta$, IL-6, IL-8, TNF-$\alpha$ and NF-kB dependent on both the significant iron-dependent decrease in the number of bacteria or viruses and the anti-inflammatory activity,

viii) anti-inflammatory activity dependent on intracellular microbial killing,

ix) activity modulating the expression of hepcidin and ferroportin,

x) activity inhibiting iron homoeostasis disorders (excess of iron in the tissues and secretions and lack of iron in circulation),

xi) activity inhibiting the increased susceptibility of the host to bacterial and viral infections induced by the accumulation of iron in tissues and secretions,

xii) prevention and treatment of iron deficiency, iron-deficiency anaemia and inflammatory anaemia,

xiii) activity repairing mucosal lesions as well as maintaining the integrity of the epithelial and nasal, intestinal and vaginal mucosal barrier whether or not linked to the persistence of iron-dependent or independent inflammation,

xiv) activity decreasing intracerebral iron accumulation,

xv) prevention activity in respect of IBDs,

xvi) prevention activity of preterm childbirth.

Furthermore, since the lactoferrin present in the mixture, thanks to sodium citrate and sodium bicarbonate, chelates iron more effectively, in addition to the numerous activities described above, the higher concentration of the form of iron-saturated lactoferrin makes it more resistant to heat and the digestion of proteolytic enzymes present in all human secretions including nasal, vaginal, anal and intestinal secretions. Furthermore, even if the proteolytic enzymes digested lactoferrin at least in part, the enzymatic digestion would separately produce the two lobes (lobe N 1-280 and lobe C 345-692) which, possessing an iron binding site per lobe, would perform the same functions as intact lactoferrin, as long as the two lobes are added together with sodium citrate and sodium bicarbonate. The two lobes as well as the intact protein can chelate in addition to iron also copper, zinc, cobalt, nickel and manganese, also necessary for bacterial development and therefore involved in the antimicrobial activity of lactoferrin which, also in the uptake of the aforementioned ions, is enhanced by sodium citrate and sodium bicarbonate.

For the uses according to the invention the formulations A and B) act as a combined preparation for a simultaneous, sequential or separate administration, which comprises, as active components, a first composition which can be administered topically, comprising the mixture with bovine lactoferrin, sodium citrate and sodium bicarbonate in the above molar ratios and a second composition for systemic oral administration, comprising the same mixture with bovine lactoferrin, sodium citrate and sodium bicarbonate always in the above molar ratios. Among the benefits obtained from the preparation according to the invention is the advantage of simultaneously resolving states of bacterial and viral infection and inflammation which occur optionally together with iron-deficiency, anaemia and inflammatory anaemia, COVID-19, IBD and preterm childbirth. Furthermore, the greater ability to chelate the iron of the mixture compared to lactoferrin alone is particularly effective in decreasing the accumulation of iron and cerebral inflammation so as to make it particularly indicated in the treatment of Alzheimer's. Currently, the known products and compositions not formulated as combined preparations for topical and oral use are not capable of achieving such an effect simultaneously.

In order to achieve an appreciable effect on both acute or chronic bacterial and viral infection and inflammation, iron accumulation, lesion repair, maintenance of epithelial and mucosal barrier integrity, iron-deficiency, anaemia and inflammatory anaemia, Alzheimer's, IBD and preterm childbirth, it is highly advantageous to follow the dosing regimen described below.

If the first topical formulation A) is in a single-dose solid form (e.g., dissolvable suppositories or tablets or intravaginal soluble granules or anal suppositories) or in solution as in a nasal spray, the mixture to be administered two or more times a day until the symptoms disappear contains a total amount of bovine lactoferrin preferably between 10 and 200 mg with sodium citrate and sodium bicarbonate added in a molar range in an amount such that the molar ratio, moles lactoferrin/(moles sodium citrate + moles sodium bicarbonate) is in the range $10^{-4}$ and $10^{-3}$, preferably $2 \times 10^{-4}$ and $7 \times 10^{-4}$.

More preferably, the aforesaid solid vaginal formulations (tablets or suppositories) or the solid anal formulations (e.g., soluble suppositories or granules) contain between 100 and 200 mg lactoferrin and are preferably administered two to several times a day.

For topical anal and intestinal administration, the formulations are preferably suppositories containing preferably 100 mg to 200 mg of lactoferrin to be administered 2 or more times a day until the disappearance of symptoms associated with infections or inflammations or lesions comprising those of the intestinal barrier typical of IBD or in the case of preterm childbirth.

If the first composition is in liquid or semi-solid multiple-dose form (for example, a solution for intranasal, intravaginal or anal administrations, sprays, gel sprays or intranasal drops or intravaginal or anal creams), the mixture contains an amount of bovine lactoferrin between 0.1-10% by weight/volume with added sodium citrate and sodium bicarbonate in such quantities as to comply with the aforementioned $10^{-4}$-$10^{-3}$ molar ratio. It is advantageous to remain, also in this case, preferably in the range 10-30 mg of bovine lactoferrin administration several times a day, until the disappearance of symptoms, as indicated above for solid compositions. If the second (systemic oral) composition administered in combination with the topical formulation is preferably in solid and single-dose form (e.g., dissolvable capsules, tablets or granules), preferably this oral formulation contains a total amount of bovine lactoferrin from 100 mg to 200 mg per capsule or tablet or granules with sodium citrate and sodium bicarbonate added in such amounts that the molar ratio of lactoferrin/moles of sodium citrate + sodium bicarbonate is in the range $10^{-4}$ and $10^{-3}$ to be administered twice or more a day until symptoms resolve, at least for one month. It is preferred that both the topical and oral administrations of the mixture object of the invention should be carried out away from meals.

The systemic oral composition administered only orally is preferably in solid, single-dose form (e.g., dissolvable capsules, tablets, or granules), containing a total amount of bovine lactoferrin comprised between an amount > 200 mg up to 500 mg with a total sodium citrate and sodium bicarbonate content between 140 and 240 mg, preferably between 170 and 200 mg, and i.e., with a total amount of each of said salts between 15 to 190 mg, preferably between 20 to 160 mg.

The amounts and topical and oral dosage administrations indicated above (defined as complete "dosage regimens"), must be considered valid and preferred aspects also in relation to the use of the mixture described above.

In addition to chelating iron more efficiently, the use of the mixture reduces more effectively, compared to lactoferrin alone, the high expression and synthesis of pro-inflammatory cytokines produced by infected epithelial cells and inhibits the production of superoxides, which, in turn, perform a pro-inflammatory action, induce cell damage and cause organ dysfunctions.

Furthermore, again by way of example, the efficacy of the mixture in the treatment of vaginal or cervicovaginal fungal infections by *Candida albicans* or bacterial infections by anaerobic bacteria such as *Gardnerella vaginalis, Prevotella* spp., *Mobiluncus* spp., *Ureaplasma, Mycoplasma,* is demonstrated using in vitro cultures of HeLa cells as a model which mimics the condition in vivo. In this type of infection, the addition of the mixture potently inhibits not only the development of *Candida* or *Gardnerella vaginalis, Prevotella* spp., *Mobiluncus* spp., *Ureaplasma, Mycoplasma,* but also the expression and synthesis of IL-6 by differently infected cells in culture. In these fungal or bacterial infections, in case of severe iron-deficiency, anaemia or inflammatory anaemia, topical administration should be combined with systemic oral administration in the treatment of vaginal mucosal infections by mycetes or bacteria and the evaluation of the effectiveness of the mixture is carried out not only by physical examination but also by counting mycetes or bacteria before and after treatment and also through clinical parameters such as the concentration of pro-inflammatory cytokines, epcidin in the blood and urine and haematological parameters such as the number of red blood cells, the concentration of haemoglobin, total serum iron and serum ferritin.

As far as viral infections are concerned, the disorders of iron homoeostasis, mainly represented by an accumulation of intra and extracellular iron and a consequent inflammatory state, are among the main predisposing factors. It follows that treatment with the mixture of the above viral infections including those associated with SARS-CoV-2 is particularly indicated as the mixture simultaneously chelates excess iron more effectively than lactoferrin alone or lactoferrin with sodium citrate alone or sodium bicarbonate alone, decreases IL-6 and other pro-inflammatory cytokines as well as prevents infection through virus-cell interaction mediated by binding to both virus proteins and cell heparan sulphate proteoglycans.

In fact, in the case of patients with COVID-19, the oral administration of the mixture containing, depending on the capsules, lactoferrin from > 200 mg up to 500 mg must be equal to 420, 600, 800 and 1,000 mg per day to be taken away from meals (two capsules per day containing different concentrations of lactoferrin). This treatment of two capsules per day containing from 210 to 500 mg lactoferrin per capsule, to be taken depending on the disease and its severity, by way of example, leads to greater inhibition of viral infection (fewer days required for negative virus response in treated patients than in untreated patients or those treated with lactoferrin alone), greater anti-inflammatory activity and greater protection from thromboembolic events.

By way of example, the mixture administered intranasally or orally to patients with Alzheimer's is more effective than lactoferrin alone in decreasing IL-6 and increasing IL-10, an anti-inflammatory cytokine, as well as inhibiting the production of superoxides as it more efficiently removes excess iron which, after treatment, is found in circulation where the haematological parameters increase.

Also by way of example, the effectiveness of the mixture in the treatment of IBD is evaluated through the protection of intestinal lesions, of any infections as well as the maintenance or restoration of the integrity of the epithelial barrier. In these diseases, in case of severe iron-deficiency, anaemia or inflammatory anaemia, topical administration should be combined with systemic oral administration and the evaluation of the effectiveness of the mixture is carried out not only by physical examination but also by clinical parameters such as the concentration of pro-inflammatory cytokines, epcidin in the blood and urine and haematological parameters such as the number of red blood cells, the concentration of haemoglobin, total serum iron and serum ferritin.

The use of the mixture in the treatment of infectious and inflammatory diseases in all human mucous membranes including nasal, vaginal and intestinal mucous membranes and the ability to increase the haematological parameters together with the treatment of Alzheimer's disease, IBD and preterm childbirth are only some of the possible therapeutic applications of the activity of simultaneously inhibiting the accumulation of iron, the expression and synthesis of pro-inflammatory cytokines and the production of superoxides.

The examples given here show that the therapeutic activity of the mixture in infections, inflammatory processes and intra and extracellular iron overload can be used against infectious and inflammatory diseases with particular attention to SARS-CoV-2 infections, intestinal infections associated with IBD, vaginal and cervicovaginal infections associated with preterm childbirth as well as to decrease cerebral iron overload and inflammation in Alzheimer's patients. In summary, the treatment by means of the mixture can be considered extremely advantageous with respect to treatments with lactoferrin alone or with other drugs currently in use, also being characterised by the absolute lack of toxicity and by the fact that multiple diseases can be simultaneously treated comprising infections, pathological inflammations, disorders of iron homoeostasis possibly associated with various diseases such as Alzheimer's, IBD and preterm childbirth. It should also be noted that, in vaginal or cervicovaginal infections, any classical antimicrobial treatment also involves the decrease of Lactobacilli, fundamental commensals in the natural defence against infections, while lactoferrin does not affect its vitality or multiplication. Furthermore, by inhibiting the synthesis of IL-6, the mixture consequently prevents the synthesis of the epcidin modulated thereby, restoring the action of ferroportin, the only protein capable of exporting iron from cells into circulation.

Furthermore, in the treatment of vaginal and intestinal mucosal lesions, the mixture leads to healing lesions in an extremely short time compared to classic therapies or lactoferrin alone.

Other features and advantages of the present invention including the treatment of preterm childbirth will better result from the description of the following preferred embodiments, comprised merely by way of non-limiting example.

Below is the procedure which demonstrates the highest efficacy of the mixture in sequestering ferric ions with respect to bovine lactoferrin in the absence of sodium citrate and sodium bicarbonate.

In vitro, the ability of lactoferrin of any origin to chelate iron is demonstrated by a series of optical density (OD) readings at 468 nm based on an extinction coefficient of 0.54 for a 1% solution of 100% iron-saturated lactoferrin, termed

EP 4 216 989 B1

holo-lactoferrin.

In detail, 10 mg of bovine lactoferrin (bLf) are dissolved in 10 ml of saline solution and the first reading is performed at 468 nm. The value obtained indicates the amount of iron contained in the commercial preparation of lactoferrin or its degree of iron saturation by the following proportion:

holo-lactoferrin (100%): 0.54 holo-lactoferrin extinction coefficient = X% (degree of sample saturation): DO of the sample at 468 nm.

Using the same procedure, 10 mg of the same commercial lactoferrin sample are dissolved in 10 ml of a 0.2 M solution of sodium citrate and the first reading is performed at 468 nm. The value obtained indicates both the amount of iron contained in the commercial preparation of lactoferrin or its degree of saturation in iron by means of the previous proportion and the possible interference by the verified sodium citrate with respect to the DO value recorded after dissolution of the lactoferrin in saline.

Using the same procedure again, 10 mg of the same commercial lactoferrin sample are dissolved in 10 ml of a 0.1 M solution of sodium bicarbonate and the first reading is performed at 468 nm. The value obtained indicates both the amount of iron contained in the commercial preparation of lactoferrin or its degree of saturation in iron by means of the previous proportion and the possible interference by the verified sodium bicarbonate with respect to the DO value recorded after dissolution of the lactoferrin in saline.

Using the same procedure, 10 mg of the same commercial sample are dissolved in 10 ml of a solution containing 0.2 M sodium citrate and 0.1 M sodium bicarbonate and the first reading is performed at 468 nm. The value obtained indicates both the amount of iron contained in the commercial preparation of lactoferrin or its degree of saturation in iron by means of the previous proportion and the possible interference of the bicarbonate and the verified sodium citrate with respect to the DO value recorded after dissolution of the lactoferrin in saline.

After the first reading of each of the 4 samples, it can be concluded that neither the saline or sodium citrate or sodium bicarbonate or the combination of sodium citrate and sodium bicarbonate interferes with the lactoferrin readings at DO 468 nm. Subsequently, 2.5 $\mu$l of ferric chloride obtained from a 0.1 M ferric chloride solution are added and mixed to each sample before reading at 468 nm.

This procedure is repeated until a sample indicates that complete iron saturation has been reached, i.e., the value of 0.540. Once this value has been reached, 25 $\mu$l of the same ferric chloride solution are added in a single addition in order to verify reaching equilibrium. The results obtained are shown in Table 1. As can be seen, regardless of the components of the mixture, the first reading values are similar and indicate an iron saturation of the commercial lactoferrin of 9.4%.

Instead, the values obtained after the addition of 2.5 $\mu$l of ferric chloride are different from those obtained when lactoferrin is added to sodium citrate (0.2 M) and sodium bicarbonate (0.1 M), indicating the greater effectiveness of the mixture in chelating iron.

The lactoferrin dissolved in saline solution, either in sodium citrate alone or in sodium bicarbonate alone, chelates iron with low efficacy (29%) and similarly in all three samples described.

Table 1. Ability to chelate ferric ion by a commercial preparation of 9.44% saturated bovine lactoferrin (bLf) (as shown by first reading) in different solutions.

| $\mu$l Ferric chloride (0.1M) | 10 mg bLf in saline solution | 10 mg bLf in 0.2M sodium citrate | 10 mg bLf in 0.1M sodium bicarbonate | 10 mg bLf in 0.2M sodium citrate and in 0.1M bicarbonate |
|---|---|---|---|---|
| 0 | 0.050 | 0.052 | 0.051 | 0.050 |
| 2.5 | 0.068 | 0.067 | 0.069 | 0.105 |
| 2.5 | 0.083 | 0.098 | 0.099 | 0.168 |
| 2.5 | 0.096 | 0.107 | 0.103 | 0.256 |
| 2.5 | 0.103 | 0.115 | 0.114 | 0.330 |
| 2.5 | 0.110 | 0.122 | 0.129 | 0.411 |
| 2.5 | 0.123 | 0.131 | 0.131 | 0.470 |
| 2.5 | 0.123 | 0.139 | 0.135 | 0.498 |
| 2.5 | 0.142 | 0.146 | 0.142 | 0.540 |
| Further addition of 25 $\mu$l ferric chloride | 0.151 | 0.157 | 0.158 | 0.540 |

Therefore, as is very evident from Table 1, bovine lactoferrin is capable of binding the ferric ion up to saturating the uptake sites 100% only in the presence of sodium citrate and bicarbonate, while in saline solution or in the presence of only sodium citrate or bicarbonate, the lactoferrin is never saturated 100%, but at most 29% indicating the poor ability to chelate iron in the above conditions.

The solution of sodium citrate and bicarbonate enhances the ability of human, bovine, equine, murine, caprine, camel, buffalo and donkey lactoferrin and recombinant human and bovine lactoferrin to chelate iron.

The molar ratio of sodium citrate to sodium bicarbonate is comprised between 1 and 3, preferably 2 as demonstrated by the above results in Table 1.

After this demonstration of the increased ability of lactoferrin to chelate iron, which underlies the multiple functions of lactoferrin, the efficacy of this mixture with bovine lactoferrin alone was compared in infections and inflammations of nasal, vaginal, anal and intestinal mucous membranes as well as in Alzheimer's, IBD, preterm childbirth and iron-deficiency, anaemia and inflammatory anaemia.

With regard to the effectiveness of concentrations of lactoferrin > 200 up to 500 mg added with sodium citrate and bicarbonate in chelating iron, the following procedure demonstrates that, even in the case of concentrations > 200 up to 500 mg, the highest effectiveness of the mixture in sequestering ferric ions is attributable to lactoferrin plus sodium citrate and sodium bicarbonate with respect to bovine lactoferrin in the absence of sodium citrate and sodium bicarbonate or added with sodium citrate alone or sodium bicarbonate alone.

As already reported in Table 1, in vitro, the ability of lactoferrin of any origin to chelate iron is demonstrated by a series of optical density (OD) readings at 468 nm based on an extinction coefficient of 0.54 for a 1% solution of 100% iron saturated lactoferrin, termed holo-lactoferrin.

In these new titrations (Table 2), the highest concentration of a new lactoferrin preparation of 500 mg was dissolved in 10 ml of the different solutions [saline, sodium citrate (160 mg/10 ml), sodium bicarbonate (70 mg/10 ml) and sodium citrate and bicarbonate at the above concentrations)]. By withdrawing 200 $\mu$l of the above solution, a concentration of 10 mg/ml of lactoferrin is obtained on which the procedure described above of the titration is based. As regards sodium citrate and bicarbonate added to 500 mg lactoferrin, their concentrations in the mixture, as already reported, correspond for sodium citrate at 160 mg and for sodium bicarbonate at 70 mg. Obviously, the concentrations reported in Table 2 are equivalent to those present in the 200 $\mu$l withdrawn for titration and dissolved in 1 ml of the different solutions. The first reading referring to 10 mg of lactoferrin dissolved in 1 ml of saline solution at 468 nm clearly indicates the amount of iron contained in the new commercial preparation of lactoferrin or its degree of iron saturation by the following ratio: holo-lactoferrin (100%): 0.54 holo-lactoferrin extinction coefficient = X% (degree of sample saturation): DO of the sample at 468 nm.

Using the same procedure, 10 mg of the same commercial lactoferrin sample are dissolved in 1 ml of a sodium citrate solution containing 3.2 mg and the first reading is performed at 468 nm. The value obtained indicates both the amount of iron contained in the commercial preparation of lactoferrin or its degree of saturation in iron by means of the previous proportion and the possible interference by the verified sodium citrate with respect to the DO value recorded after dissolution of the lactoferrin in saline.

Using the same procedure again, 10 mg of the same commercial lactoferrin sample are dissolved in 1 ml of a solution containing 1.4 mg sodium bicarbonate and the first reading is performed at 468 nm. The value obtained indicates both the amount of iron contained in the commercial preparation of lactoferrin or its degree of saturation in iron by means of the previous proportion and the possible interference by the verified sodium bicarbonate with respect to the DO value recorded after dissolution of the lactoferrin in saline.

Using the same procedure, 10 mg of the same commercial sample are dissolved in 1 ml of a solution containing 3.2 mg sodium citrate and 1.4 mg sodium bicarbonate and the first reading is performed at 468 nm. The value obtained indicates both the amount of iron contained in the commercial preparation of lactoferrin or its degree of saturation in iron by means of the previous proportion and the possible interference of the bicarbonate and the verified sodium citrate with respect to the DO value recorded after dissolution of the lactoferrin in saline.

After the first reading of each of the 4 samples, it can be concluded that neither the saline solution or sodium citrate or sodium bicarbonate or the combination of sodium citrate and sodium bicarbonate interferes with the lactoferrin readings at DO 468 nm. 2.5 $\mu$l of ferric chloride (0.01 M) are then added and mixed to each sample before reading at 468 nm.

This procedure is repeated until a sample indicates that complete iron saturation has been reached, i.e., the value of 0.540. Once this value has been reached, 25 $\mu$l of the same ferric chloride solution are added in a single addition in order to verify reaching equilibrium. The results obtained with this new commercial preparation are shown in Table 2. As can be seen, regardless of the components of the mixture, the first reading values are similar for all conditions and indicate an iron saturation of the commercial lactoferrin of 13%.

Instead, the values obtained after the addition of ferric chloride (2.5 $\mu$l) to lactoferrin in the presence of sodium citrate and sodium bicarbonate are different from those obtained when lactoferrin is added to sodium citrate alone or sodium bicarbonate alone, indicating the greater efficacy of the mixture in chelating iron. The lactoferrin dissolved in saline solution, either in sodium citrate alone or in sodium bicarbonate alone, chelates iron with low efficacy and similarly in all three samples described. In fact, the maximum value of iron uptake is equal to 34%.

Table 2. Ability to chelate ferric ion by a commercial preparation of 13% saturated bovine lactoferrin (bLf) (as shown by first reading) in different solutions.

| µl FERRIC CHLORIDE (0.01M) | 10 mg bLf in 1 ml saline solution (SF) OPTICAL DENSITY | 10 mg bLf + 1.4 mg of sodium bicarbonate OPTICAL DENSITY | 10 mg bLf + 3.2 mg of sodium citrate OPTICAL DENSITY | 10 mg bLf + 1.4 mg sodium bicarbonate + 3.2 mg sodium citrate OPTICAL DENSITY |
|---|---|---|---|---|
| 0 | 0.069 | 0.072 | 0.070 | 0.071 |
| 2.5 | 0.110 | 0.094 | 0.091 | 0.144 |
| 2.5 | 0.132 | 0.117 | 0.116 | 0.226 |
| 2.5 | 0.140 | 0.136 | 0.126 | 0.313 |
| 2.5 | 0.145 | 0.142 | 0.136 | 0.384 |
| 2.5 | 0.151 | 0.148 | 0.144 | 0.408 |
| 2.5 | 0.153 | 0.155 | 0.151 | 0.434 |
| 2.5 | 0.155 | 0.167 | 0.155 | 0.470 |
| 2.5 | 0.158 | 0.178 | 0.158 | 0.488 |
| 2.5 | 0.160 | 0.180 | 0.160 | 0.508 |
| 2.5 | 0.163 | 0.184 | 0.162 | 0.516 |
| 2.5 | 0.167 | 0.187 | 0.165 | 0.520 |
| 2.5 | 0.170 | 0.187 | 0.167 | 0.534 |
| 25 | **0.170** | **0.187** | **0.168** | **0.534** |

**Example 1. Activity of the mixture against bacterial or fungal vaginal infections**

Vaginal infections associated with bacteria or fungi can be treated by intravaginal administration of lactoferrin alone. Despite the good efficacy demonstrated by lactoferrin against vaginal infections of both bacterial and fungal origin, relapses were observed within 2 or 3 months. The intravaginal application of the mixture administered in tablets or suppositories including those with slow release does not induce relapses and the number of bacteria *(Gardnerella vaginalis, Prevotella* spp., *Mobiluncus* spp., *Ureaplasma, Mycoplasma)* or Candida spp, determined before and after 7 days of treatment appears to be significantly lower if the mixture is used. The vaginal tablets or suppositories contain 100 mg lactoferrin, 0.84 mmol sodium citrate dihydrate and 0.42 mmol sodium bicarbonate and should be applied 2 to 4 times daily. The mixture contained in the vaginal tablets or suppositories, despite having the same concentration of lactoferrin and despite being applied the same number of times a day, is more effective than the preparation of lactoferrin which does not contain sodium bicarbonate and sodium citrate since it chelates iron with greater effectiveness and therefore has a greater antimicrobial activity. In the event of iron-deficiency, anaemia or inflammatory anaemia, proceed by combining intravaginal topical therapy with the mixture also with systemic oral therapy also with the mixture. Systemic oral administration of lactoferrin alone requires 20 days of therapy to reach the values obtained from the mixture in 4-10 days, depending on the severity of the infection.

**Example 2. Activity of the mixture against infections due to Herpesvirus type 2, the aetiological agent of genital herpes.**

The cellular monolayer (HeLa cells) is infected with Herpesvirus type 2 in the presence of 100 µg/ml lactoferrin alone or of the mixture containing 100 µg/ml lactoferrin, 0.00084 mmol of sodium citrate dihydrate and 0.00042 mmol of sodium bicarbonate or in the absence of lactoferrin. The monolayer is then incubated at 37°C for 48 h. After this period, the viral concentration decreases and the level of IL-6 is tested by an enzyme immunoassay (ELISA). The results referring to IL-6 are shown below in Table 3.

Table 3. Synthesis of IL-6 by HeLa cells infected with Herpesvirus type 2 in the absence of lactoferrin, in the presence of lactoferrin or the mixture.

| | IL-6 pg/ml | | |
|---|---|---|---|
| Conditions | No lactoferrin | Addition of lactoferrin | Addition of the mixture |
| Uninfected cells | 37 | 35 | 35 |
| Infected cells | 380 | 126 | 57 |

As the results show, the mixture decreases the concentration of IL-6 more than lactoferrin alone.

*In vivo applications:* administration of intravaginal tablets or suppositories containing the mixture with 100 mg of lactoferrin, 0.84 mmol of sodium citrate dihydrate and 0.42 mmol of sodium bicarbonate from 2 to 4 times per day depending on the severity of the disease leads to healing of the viral infection and any lesions after about 4-7 days of treatment compared to the two weeks required by lactoferrin alone at the same concentration and applied the same number of times per day. If the genital infection is associated with iron-deficiency, anaemia or inflammatory anaemia, the mixture containing 100 mg of lactoferrin, 0.84 mmol of sodium citrate dihydrate and 0.42 mmol of sodium bicarbonate (citrate and bicarbonate moles are not reported) is administered orally systemically at least twice daily far from meals for 15 days, after which the haematological values are restored.

## Example 3: Efficacy of the mixture against SARS-CoV-2 infection

Before infecting the cellular monolayer (Vero E6), SARS-CoV-2 at a multiplicity of infection (MOI) of 0.01 is pre-incubated in the presence of 100 μg/ml lactoferrin alone or of the mixture containing 100 μg/ml lactoferrin, 0.00084 mmol of sodium citrate dihydrate and 0.00042 mmol of sodium bicarbonate for 1 h at 37 °C or in the absence of lactoferrin. Subsequently the SARS-CoV-2 infected monolayer is incubated at 37°C for 48 h. After 48h, the monolayer was washed, fixed with 5% formaldehyde for 10 min at room temperature and stained with 1% violet crystal for 5 min. The number of plaques was determined after repeated washing. The data obtained showed that the plaque inhibition operated by the mixture was, with respect to the control, equal to 4 logs and greater than the inhibition operated by the lactoferrin alone which was equal to 2 logs (Table 4).

Table 4: Number of plaques in the presence of lactoferrin (100 μg/ml) or the mixture determined on Vero E2 monolayer infected with 0.01 MOI of SARS-CoV-2

| | Infected monolayer in the absence of lactoferrin | Infected monolayer In the presence of lactoferrin | Infected monolayer in the presence of the mixture |
|---|---|---|---|
| Plaque forming units | $5 \times 10^5$ | $1 \times 10^3$ | $1 \times 10$ |

From the data in Table 4 it is clear that the mixture has greater antiviral activity than lactoferrin alone.

## Example 4: Effectiveness of the mixture in Alzheimer's patients

Since iron overload plays a pivotal role in the development and progression of Alzheimer's, in recent years the use of iron chelators has received a great deal of attention. However, the entry of almost all drugs into the brain is complicated by the presence of the blood brain barrier (BBB). In particular, iron chelators such as deferoxamine (DFO) show poor bioavailability and induce some side effects such as neurotoxicity if treatment is prolonged in the long term. In addition to these adverse effects, it is important to point out that, while it is clear how DFO performs a specific iron-chelating action, its specific action against IL-6 and iron protein expression in Alzheimer's has never been investigated.

Unlike the iron chelators known so far, lactoferrin is emerging as an important natural compound with a potent anti-inflammatory activity especially against IL-6 and consequently able to down-regulate epcidin and up-regulate ferroportin. Therefore, lactoferrin not only sequesters two iron atoms per molecule but, especially by decreasing IL-6, epcidin and iron accumulation as well as increasing ferroportin synthesis, removes the root cause of the harmful iron homoeostasis disorders in Alzheimer's. Obviously, the mixture, being more effective in chelating iron than lactoferrin alone and being able to cross the BBB due to the presence of its receptors (LfR) on the membrane of the endothelial cells (Huang et al 2006), can exercise its multiple functions. It is also interesting to note that, in pathological conditions such as Alzheimer's, an increase in LfR expression is observed on micro-vessels and neurons so that lactoferrin is rapidly taken up by its own receptor and is available in the brain.

Treatment with the intranasal mixture of the invention (lactoferrin 10 mg, 0.084 sodium citrate dihydrate moles and 0.042 bicarbonate moles 3 times a day) combined with treatment with the oral formulation mixture (100 mg 0.84 sodium citrate dihydrate moles and 0.42 sodium bicarbonate moles twice a day for three months) was particularly effective in reducing serum IL-6 and increasing serum IL-10 without causing the undesirable effects induced by other treatments.

**Example 5. Activity of the mixture against bacterial, fungal or viral anal or intestinal infections**

Also in the case of anal or intestinal infections associated with bacteria or fungi or viruses, lactoferrin alone has been shown to be effective even if the complete eradication of pathogenic microorganisms cannot be achieved through twice-daily administration of suppositories containing 100 mg of lactoferrin. The twice daily anal application of the suppositories containing the mixture (lactoferrin 100 mg, sodium citrate dihydrate 0.84 mmol and sodium bicarbonate 0.42 mmol) decreases the number of pathogenic microorganisms more significantly with respect to lactoferrin alone. In the case of iron-deficiency, anaemia or inflammatory anaemia, topical anal therapy with the mixture is also associated with systemic oral therapy with the mixture. Oral administration of lactoferrin alone requires 30 days of therapy to reach the values obtained from the mixture in only 15 days.

Also, the mixture repairs lesions due to inflammation in IBDs more effectively than lactoferrin alone. In subjects in which bowel disease is associated with iron-deficiency, anaemia and inflammatory anaemia, systemic oral administration of the mixture (100 mg lactoferrin + 0.84 mmol sodium citrate dihydrate+ 0.42 mmol sodium bicarbonate) twice daily away from meals is more effective in restoring haematological parameters than lactoferrin alone (100 mg twice daily away from meals).

**Example 6: Effectiveness of the mixture in the treatment of preterm childbirth**

In pregnancy, iron-deficiency, anaemia, and inflammatory anaemia are associated with preterm childbirth and increase its incidence. It follows that in order to prevent this disease, pregnant women should be treated with the lactoferrin-containing mixture (100 mg twice daily) by systemic oral use in order to restore haematological values. Despite this therapy, in some women the symptoms of preterm childbirth (the onset of uterine contractions) associated with high levels of IL-6, prostaglandins and shortening of the cervix are observed due to the occurrence of vaginal infections and significant inflammatory phenomena. The intravaginal administration of the mixture (100 mg lactoferrin, 0.84 mmol of sodium citrate dihydrate and 0.42 mmol of sodium bicarbonate) in dissolvable tablets or suppositories at least every 6-8 h, having a higher antimicrobial activity than lactoferrin alone (100 mg), has a greater effectiveness in counteracting preterm childbirth. The results with better efficacy are shown in Table 5.

Table 5. Intravaginal treatment with lactoferrin or the mixture in women with risk of preterm childbirth

| Subjective symptomatology and objective parameters (mean values) before treatment | Subjective symptomatology and objective parameters (mean values) after 7 days of treatment with lactoferrin every 8 h | Subjective symptomatology and objective parameters (average values) after 7 days of treatment with the mixture every 8 h |
|---|---|---|
| 2 -5 uterine contractions /day | 2-3 uterine contractions/day | 1-2 uterine contractions/day |
| IL-6 (pg/ml) **1,425** | IL-6 (pg/ml) **854** | IL-6 (pg/ml) **187** |
| Fetal fibronectin (ng/ml) **>50** | Fetal fibronectin (ng/ml) **>50** | Fetal fibronectin (ng/ml) **<50** |
| Prostaglandin E2 (pg/ml) **569** | Prostaglandin E2 (pg/ml) **298** | Prostaglandin E2 (pg/ml) **43** |
| Fetal prostaglandin (pg/ml) **1,234** | Fetal prostaglandin (pg/ml) **832** | Fetal prostaglandin (pg/ml) **320** |

From the results reported and from the inhibition of the further shortening of the cervix in women treated with the intravaginal mixture, it is clear that the mixture, having a greater antibacterial activity based on its greater ability to chelate iron, subtracting it from bacteria, is significantly more effective in the treatment of preterm childbirth as demonstrated by subjective and objective parameters.

**Example 7. Activity of the mixture in the treatment of iron deficiency or anaemia**

In the treatment of iron deficiency or anaemia, subjects must take capsules containing 100 mg of lactoferrin two or three times a day away from meals and for a duration of at least one month or until the disease resolves. Table 6 compares the haematological parameters (mean values) obtained in 77 patients enrolled with iron deficiency or anaemic patients whose study included 36 patients treated with capsules containing 100 mg of lactoferrin and 36 treated with capsules containing 100 mg of lactoferrin added with sodium citrate dihydrate (0.84 mmol) and sodium bicarbonate (0.42 mmol) twice a day away from meals for 30 days.

Table 6. Observational study in 72 patients with iron deficiency and anaemia, of which 36 were treated twice a day for one month with capsules containing 100 mg of lactoferrin and 36 were treated twice a day with capsules containing 100 mg of lactoferrin plus sodium citrate dihydrate (0.84 mmol) and sodium bicarbonate (0.42 mmol).

| | | After 30 days of treatment with | |
|---|---|---|---|
| Haematological Parameters (mean values) | Before treatment | Lactoferrin 2 times daily | Mixture 2 times daily |
| Haemoglobin (g/dL) | 10.8±0.2 | 11.2±0.2 | 12.1±0.2 |
| Red blood cells (x$10^3$) | 3,900±35 | 4,027±20 | 4,550±10 |
| Total serum iron ($\mu$g/dL) | 25±7 | 50±8 | 85±10 |
| Ferritin Serum (ng/ml) | 13±2 | 19±4 | 24±3 |
| Haematocrit (%) | 28±6 | 32±5 | 35±3 |

**Example 8. Activity of the mixture in the treatment of inflammatory anaemia**

In the treatment of inflammatory anaemia, subjects must take capsules containing 100 mg of lactoferrin three or four times daily away from meals and for a duration of at least one month or until the disease resolves.

Table 7 compares the haematological parameters (mean values), the concentration of IL-6 in the serum of 60 patients enrolled with inflammatory anaemia whose study included 26 treated with capsules containing 100 mg of lactoferrin and 28 treated with capsules containing 100 mg of lactoferrin plus sodium citrate dihydrate (0.84mmol) and sodium bicarbonate (0.42mmol) three times a day away from meals for 30 days.

Table 7: Haematological parameters in patients with inflammatory anaemia treated with lactoferrin or the mixture

| | | After 30 days of treatment with | |
|---|---|---|---|
| Haematological parameters (mean values) | Before treatment | Lactoferrin twice daily | Mixture twice daily |
| Haemoglobin (g/dL) | 10.3±0.3 | 10.9±0.2 | 12.1±0.3 |
| Red blood cells (x$10^3$) | 3,700±39 | 4,045±28 | 4,580±12 |
| Total serum iron ($\mu$g/dL) | 21±8 | 45±9 | 91±14 |
| Ferritin Serum (ng/ml) | 10±3 | 15±2 | 26±5 |
| IL-6 (pg/ml) | 45±13 | 33±9 | 9±5 |

**Bibliography**

Beddek, A.J.; Schryvers, A.B. The lactoferrin receptor complex in Gram negative bacteria. Biometals 2010, 23,377-386.

Calvani F, Cutone A, Lepanto MS, Rosa L, Valentini V, Valenti P. Efficacy of bovine lactoferrin in the post-surgical treatment of patients suffering from bisphosphonate-related osteonecrosis of the jaws: an open-label study. Biometals. 2018 Jun;31(3):445-455.

Campione E, Cosio T, Rosa L, Lanna C, Di Girolamo S, Gaziano R, Valenti P, Bianchi L. Lactoferrin as Protective Natural Barrier of Respiratory and Intestinal Mucosa against Coronavirus Infection and Inflammation. Int J Mol Sci. 2020 Jul 11;21(14):4903

Mancinelli R, Rosa L, Cutone A, Lepanto MS, Franchitto A, Onori P, Gaudio E, Valenti P. Viral Hepatitis and Iron Dysregulation: Molecular Pathways and the Role of Lactoferrin. Molecules. 2020 Apr 24;25(8):1997

Mohamed WA, Schaalan MF. Antidiabetic efficacy of lactoferrin in type 2 diabetic pediatrics; controlling impact on PPAR-$\gamma$, SIRT-1, and TLR4 downstream signaling pathway.DiabetolMetabSyndr. 2018 Dec 4;10:89.

Paesano R, Natalizi T, Berlutti F, Valenti P. Body iron delocalization: the serious drawback in iron disorders in both developing and developed countries. Pathog Glob Health. (2012)

Petrik, M.; Zhai, C.; Haas, H.; Decristoforo, C. Siderophores for molecular imaging applications.Clin. Transl. Imaging 2017, 5, 15-27.

Puddu, P.; Latorre, D.; Carollo,M.; Catizone, A.; Ricci, G.; Valenti, P.; Gessani, S.
Bovine lactoferrin counteracts Toll-like receptor mediated activation signals in antigen presenting cells. PLoS ONE 2011, 6, e22504.

Rosa L, Cutone A, Lepanto MS, Paesano R, Valenti P. Lactoferrin: A Natural Glycoprotein Involved in Iron and Inflammatory Homeostasis.Int J Mol Sci. 2017 Sep 15;18(9).

Rosa L, Cutone A, Lepanto MS, Scotti MJ, Conte MP, Paesano R, Valenti P. Physicochemical properties influence the functions and efficacy of commercial bovine lactoferrins.Biometals. 2018 Jun;31(3):301-312.

Valenti P, Antonini G. Lactoferrin: an important host defence against microbial and viral attack. Cell Mol Life Sci. 2005 Nov;62(22):2576-87.

XP 002788307: Adlerova et al "Lactoferrin a review" Veterinarni Medicina, 2008-09-15, Ceska Akademie Zemedelskych Ved,Czech Academy of Agricultural Sciences, CZ vol.53 no.9 pages 457-468-

XP 002325824 Strate Van Der B W A, Et A 2001-12-01 "Antiviral activities of lactoferrin" Vol.:52, Nr.:3, Pages:225-239XP 0055744173 Valenti P. et al "Role of Lactobacilli and Lactoferrin in the Mucosal Cervicovaginal Defense" 2018-03-01 Frontiers in Immunology. Volume 9-

XP 0055808182 Lepanto M.S. et al "Lactoferrin in Aseptic and Septic Inflammation" 2019-04-03 Molecules Vol.:24, Nr.:7, Pages:1323.

XP 003019976 Nonnecke B J, et al, 1984-01-01 Indian Journal Of Dairy Science. Vol.:67, Nr.:3, Pages:606 - 613,

XP 005576575 Jianshe Lang,et al "Inhibition of SARS Pseudovirus Cell Entry by Lactoferrin Binding to Heparan Sulfate" 2011-08-01 PLoS ONE, 2011-08-01, Public Library of Science Vol.:6, Nr.:8, Pages:e23710.

XP 0055872688 Mirabelli Carmen,et al "Morphological Cell Profiling of SARS-CoV-2 Infection Identifies Drug Repurposing" 2020-06-10 bioRxiv, Pages:1-36.

XP0023475768 Mazurier J Et Al, "Comparative study of the iron-binding properties of human transferrins II. Electron paramagnetic resonance of mixed metal complexes of human lactotransferrin", Biochimica Et Biophysica Acta. Protein Structure And Molecular Enzymology, Elsevier, Amsterdam; NL, vol. 745, no. 1.

**Claims**

1. A topical nasal, vaginal or anal formulation A) comprising as active ingredient a mixture containing lactoferrin, sodium citrate, sodium bicarbonate in a molar ratio

$$\frac{(lactoferrin\ moles)}{(sodium\ citrate\ moles + sodium\ bicarbonate\ moles)}$$

comprised between $10^{-4}$ and $10^{-3}$, preferably between $2 \times 10^{-4}$ and $7 \times 10^{-4}$, for use in the treatment

or prevention of bacterial and viral infections which colonise the epithelia at the nasal, vaginal, cervicovaginal, anal and intestinal mucosal levels in order to:

- decrease the number of pathogenic bacteria and viruses,
- inhibit biofilm formation, microbial ability to adhere and invade,
- inhibit or decrease the inflammatory process caused by infections, said inflammatory process possibly being associated with iron deficiency, anaemia or inflammatory anaemia or IBD,
- decrease intracerebral iron overload as occurs in Alzheimer's,
- facilitate the repair of lesions,
- prevent preterm childbirth induced by vaginal infections and/or inflammations optionally associated with iron deficiency, anaemia or inflammatory anaemia,

said topical formulation A) being administered only nasally, intravaginally to the nasal, vaginal and cervicovaginal mucous membranes or topically intra-anally to the anal and intestinal mucous membranes, in the absence of iron homoeostasis disorders, or

said topical formulation A) being administered nasally, vaginally or anally in combination with a systemic oral formulation B), containing said mixture wherein the lactoferrin, sodium citrate and sodium bicarbonate are present in the same molar ratio range as above, when said mucosal infections and the inflammatory process thereof manifest with iron deficiency, anaemia or inflammatory anaemia, or

the aforementioned systemic oral formulation B) is administered in combination with said nasal topical formulation A), when cerebral iron overload and the inflammatory processes thereof are associated with Alzheimer's.

2. The topical formulation A) and when needed combined with the oral formulation B) for use according to claim 1, wherein the following effects are obtained simultaneously:

> significant inhibition of the multiplication of pathogenic bacteria and viruses
> inhibition of biofilm formation
> inhibition of bacterial and viral adhesiveness
> inhibition of pathogenic bacteria and viruses entering cells
> inhibition of pro-inflammatory cytokine synthesis
> prevention and treatment of IBD
> restoration of iron homoeostasis
> inhibition of intracerebral iron overload
> restoration of the physiological values of haemoglobin, number of red blood cells, total serum iron, serum ferritin, haematocrit percentage
> protection for vaginal or intestinal lesions
> prevention and treatment of preterm childbirth.

3. The topical formulation A) and when needed combined with the oral formulation B) for use according to claim 1 or 2, wherein acute or chronic infections are selected from bacterial infections caused by bacteria in planktonic form or in optional or obligatory, adhesion or intracellular biofilms.

4. The topical formulation A) and when needed combined with the oral formulation B) for use according to any one of claims 1-3, where the treatment of iron deficiency, anaemia, or inflammation anaemia with said systemic oral formulation B) induces restoring of the physiological iron distribution among tissues and secretions and the circulation.

5. The topical formulation A) and when needed combined with the oral formulation B) for use according to any one of claims 1-4 wherein the lactoferrin in the topical formulation A) and in the oral formulation B) in amounts comprised between 10 and 200mg.

6. The topical formulation A) and when needed combined with the oral formulation B) for use according to any one of claims 1- 5, wherein the lactoferrin has a degree of iron saturation from 0% to 100%, preferably from 0% (limit included) to 100% (limit not included), more preferably from 0% to 20% (limit included) saturation.

7. The topical formulation A) and when needed combined with the oral formulation B)for use according to any one of claims 1-6, , wherein the saturation degree is obtained with one or more Fe (III), Zn, Cu and Mn and wherein the lactoferrin is selected from one or more intact lactoferrin, the N lobe, the C lobe and the iron-binding site-containing sequences.

8. The topical formulation A) and when needed combined with the oral formulation B), for use according to any one of claims 1-7, , wherein said nasal topical formulation is in drop form or vaginal is in spray, tablets, ova, and granule form, preferably in dissolvable tablets or ova form, or anal topical formulation is in form of dissolvable suppositories or granules.

9. The topical formulation A) and when needed combined with the oral formulation B) for use according to any one of claims 1-8, wherein by said administration, intravaginally, intra-anally from 10 to 200mg lactoferrin is administered two or more times daily until resolution of the pathology.

10. The topical formulation A) and when needed combined with the oral formulation B) for use according to any one of claims 1-7, being administered by the aforementioned intranasal route, several times a day in solutions containing the lactoferrin in concentrations comprised between 0.1 and 10% by weight/volume and the lactoferrin is preferably contained in said solutions in amounts comprised between 10 and 30 mg.

11. The topical formulation A) and when needed combined with the oral formulation (B) for use according to any one of claims 1-7, wherein when said vaginal formulations are selected from tablets or ova or the solid anal formulations, is selected from soluble suppositories or granules contain between 100 and 200 mg of lactoferrin and are preferably administered at least twice a day.

12. The topical formulation A) when needed combined with the oral formulation B) for use according to any one of claims 1-10 , wherein the oral formulation contains a total amount of bovine lactoferrin from 100 mg to 200 mg to be administered two or more times a day for at least one month.

13. An oral composition comprising as active ingredient a mixture of lactoferrin in amounts greater than 200 mg up to 500 mg, preferably between 250 and 500mg, and a total content of sodium citrate and sodium bicarbonate comprised between 140 and 240 mg, preferably between 170 and 200mg in which each of said salts is present in an amount comprised between 15 and 190 mg preferably between 20 and 160 mg for use in the treatment and prevention of viral infections by DNA, RNA, enveloped or naked viruses.

14. The oral composition for use according to claim 13, wherein the virus is SARS-CoV-2 or other viruses causing intracellular and extracellular iron overload and an inflammatory storm.

**Patentansprüche**

1. Eine topische nasale, vaginale oder anale Formulierung A), umfassend als Wirkstoff eine Mischung aus Lactoferrin, Natriumcitrat, Natriumbicarbonat in einem Molverhältnis

$$\frac{\text{(Lactoferrin-Mole)}}{\text{(Natriumcitrat-Mole + Natriumbicarbonat-Mole)}},$$

das zwischen $10^{-4}$ und $10^{-3}$ liegt, vorzugsweise zwischen $2 \times 10^{-4}$ und $7 \times 10^{-4}$, zur Verwendung bei der Behandlung oder Prävention von bakteriellen und viralen Infektionen, die die Epithelien der nasalen, vaginalen, zervikovaginalen, analen und intestinalen Schleimhäute besiedeln, um:

- die Anzahl pathogener Bakterien und Viren zu verringern,
- die Biofilmbildung, die mikrobielle Fähigkeit zur Adhäsion und Invasion zu hemmen,
- den durch Infektionen verursachten Entzündungsprozess zu hemmen oder zu verringern, wobei der Entzündungsprozess möglicherweise mit Eisenmangel, Anämie oder inflammatorischer Anämie oder IBD assoziiert ist,
- die intrazerebrale Eisenüberladung, wie sie bei Alzheimer auftritt, zu verringern,
- die Reparatur von Läsionen zu erleichtern,
- eine durch vaginale Infektionen und/oder Entzündungen ausgelöste Frühgeburt zu verhindern, die optional mit Eisenmangel, Anämie oder inflammatorischer Anämie assoziiert sind, wobei die topische Formulierung A) nur nasal, intravaginal an die nasalen, vaginalen und zervikovaginalen Schleimhäute oder topisch intraanal an die analen und intestinalen Schleimhäute in Abwesenheit von Eisenhomöostasestörungen verabreicht wird, oder wobei die topische Formulierung A) nasal, vaginal oder anal in Kombination mit einer systemischen oralen Formulierung B) verabreicht wird, die die Mischung enthält, wobei das Lactoferrin, das Natriumcitrat und das Natriumbicarbonat im gleichen oben genannten Molverhältnisbereich vorliegen, wenn sich die Schleimhautinfektionen und deren Entzündungsprozess mit Eisenmangel, Anämie oder inflammatorischer Anämie manifestieren, oder wobei die vorgenannte systemische orale Formulierung B) in Kombination mit der nasalen topischen Formulierung A) verabreicht wird, wenn die zerebrale Eisenüberladung und deren Entzündungsprozesse mit Alzheimer assoziiert sind.

2. Die topische Formulierung A) und bei Bedarf kombiniert mit der oralen Formulierung B) zur Verwendung nach Anspruch 1, wobei die folgenden Wirkungen gleichzeitig erzielt werden:

- signifikante Hemmung der Vermehrung von pathogenen Bakterien und Viren
- Hemmung der Biofilmbildung
- Hemmung der bakteriellen und viralen Adhäsivität

○ Hemmung des Eindringens von pathogenen Bakterien und Viren in Zellen
○ Hemmung der pro-inflammatorischen Zytokinsynthese
○ Prävention und Behandlung von IBD
○ Wiederherstellung der Eisenhomöostase
○ Hemmung der intrazerebralen Eisenüberladung
○ Wiederherstellung der physiologischen Werte von Hämoglobin, Anzahl der roten Blutkörperchen, Gesamteisen im Serum, Serumferritin, Hämatokrit-Prozentsatz
○ Schutz für vaginale oder intestinale Läsionen
○ Prävention und Behandlung von Frühgeburten.

3. Die topische Formulierung A) und bei Bedarf kombiniert mit der oralen Formulierung B) zur Verwendung nach Anspruch 1 oder 2, wobei akute oder chronische Infektionen aus bakteriellen Infektionen ausgewählt sind, die durch Bakterien in planktonischer Form oder in optionalen oder obligatorischen, adhäsiven oder intrazellulären Biofilmen verursacht werden.

4. Die topische Formulierung A) und bei Bedarf kombiniert mit der oralen Formulierung B) zur Verwendung nach einem der Ansprüche 1-3, wobei die Behandlung von Eisenmangel, Anämie oder inflammatorischer Anämie mit der systemischen oralen Formulierung B) eine Wiederherstellung der physiologischen Eisenverteilung zwischen Geweben und Sekreten sowie dem Kreislauf bewirkt.

5. Die topische Formulierung A) und bei Bedarf kombiniert mit der oralen Formulierung B) zur Verwendung nach einem der Ansprüche 1-4, wobei das Lactoferrin in der topischen Formulierung A) und in der oralen Formulierung B) in Mengen zwischen 10 und 200 mg enthalten ist.

6. Die topische Formulierung A) und bei Bedarf kombiniert mit der oralen Formulierung B) zur Verwendung nach einem der Ansprüche 1-5, wobei das Lactoferrin einen Eisensättigungsgrad von 0 % bis 100 % aufweist, vorzugsweise von 0 % (einschließlich) bis 100 % (ausschließlich), besonders bevorzugt von 0 % bis 20 % (einschließlich) Sättigung.

7. Die topische Formulierung A) und bei Bedarf kombiniert mit der oralen Formulierung B) zur Verwendung nach einem der Ansprüche 1-6, wobei der Sättigungsgrad mit einem oder mehreren von Fe (III), Zn, Cu und Mn erreicht wird und wobei das Lactoferrin aus einem oder mehreren von intaktem Lactoferrin, dem N-Lappen, dem C-Lappen und den die Eisenbindungsstelle enthaltenden Sequenzen ausgewählt ist.

8. Die topische Formulierung A) und bei Bedarf kombiniert mit der oralen Formulierung B) zur Verwendung nach einem der Ansprüche 1-7, wobei die nasale topische Formulierung in Tropfenform, die vaginale Formulierung in Spray-, Tabletten-, Ovula- und Granulatform, vorzugsweise in Form von löslichen Tabletten oder Ovula, oder die anale topische Formulierung in Form von löslichen Zäpfchen oder Granulaten vorliegt.

9. Die topische Formulierung A) und bei Bedarf kombiniert mit der oralen Formulierung B) zur Verwendung nach einem der Ansprüche 1-8, wobei bei dieser Verabreichung 10 bis 200 mg Lactoferrin intravaginal, intraanal zwei- oder mehrmals täglich bis zum Abklingen der Pathologie verabreicht werden.

10. Die topische Formulierung A) und bei Bedarf kombiniert mit der oralen Formulierung B) zur Verwendung nach einem der Ansprüche 1-7, wobei sie über den vorgenannten intranasalen Weg mehrmals täglich in Lösungen verabreicht wird, die das Lactoferrin in Konzentrationen zwischen 0,1 und 10 % Gewicht/Volumen enthalten, und das Lactoferrin vorzugsweise in den Lösungen in Mengen zwischen 10 und 30 mg enthalten ist.

11. Die topische Formulierung A) und bei Bedarf kombiniert mit der oralen Formulierung (B) zur Verwendung nach einem der Ansprüche 1-7, wobei die vaginalen Formulierungen, wenn sie aus Tabletten oder Ovula ausgewählt sind, oder die festen analen Formulierungen, wenn sie aus löslichen Zäpfchen oder Granulaten ausgewählt sind, zwischen 100 und 200 mg Lactoferrin enthalten und vorzugsweise mindestens zweimal täglich verabreicht werden.

12. Die topische Formulierung A), bei Bedarf kombiniert mit der oralen Formulierung B), zur Verwendung nach einem der Ansprüche 1-10, wobei die orale Formulierung eine Gesamtmenge an Rinder-Lactoferrin von 100 mg bis 200 mg enthält, die zwei- oder mehrmals täglich für mindestens einen Monat zu verabreichen ist.

13. Eine orale Zusammensetzung, umfassend als Wirkstoff eine Mischung aus Lactoferrin in Mengen von mehr als 200 mg bis zu 500 mg, vorzugsweise zwischen 250 und 500 mg, und einem Gesamtgehalt an Natriumcitrat und Natriumbicarbonat zwischen 140 und 240 mg, vorzugsweise zwischen 170 und 200 mg, wobei jedes der Salze in einer Menge zwischen 15 und 190 mg, vorzugsweise zwischen 20 und 160 mg, vorliegt, zur Verwendung bei der Behandlung und Prävention von viralen Infektionen durch DNA-, RNA-, umhüllte oder unbehüllte Viren.

**14.** Die orale Zusammensetzung zur Verwendung nach Anspruch 13, wobei das Virus SARS-CoV-2 oder andere Viren ist, die eine intrazelluläre und extrazelluläre Eisenüberladung und einen Entzündungssturm verursachen.

**Revendications**

**1.** Une formulation topique nasale, vaginale ou anale A) comprenant comme principe actif un mélange contenant de la lactoferrine, du citrate de sodium, du bicarbonate de sodium dans un rapport molaire

$$\frac{(\text{moles de lactoferrine})}{(\text{moles de citrate de sodium} + \text{moles de bicarbonate de sodium})}$$

compris entre $10^{-4}$ et $10^{-3}$, de préférence entre $2 \times 10^{-4}$ et $7 \times 10^{-4}$, pour une utilisation dans le traitement ou la prévention d'infections bactériennes et virales qui colonisent les épithéliums au niveau des muqueuses nasales, vaginales, cervico-vaginales, anales et intestinales afin de :

- ◦ diminuer le nombre de bactéries et de virus pathogènes,
- ◦ inhiber la formation de biofilm, la capacité microbienne à adhérer et à envahir,
- ◦ inhiber ou diminuer le processus inflammatoire provoqué par les infections, ledit processus inflammatoire étant éventuellement associé à une carence en fer, une anémie ou une anémie inflammatoire ou une MICI (Maladie Inflammatoire Chronique de l'Intestin),
- ◦ diminuer la surcharge en fer intracérébrale telle qu'elle se produit dans la maladie d'Alzheimer,
- ◦ faciliter la réparation des lésions,
- ◦ prévenir l'accouchement prématuré induit par des infections et/ou des inflammations vaginales éventuellement associées à une carence en fer, une anémie ou une anémie inflammatoire, ladite formulation topique A) étant administrée uniquement par voie nasale, par voie intravaginale sur les muqueuses nasales, vaginales et cervico-vaginales ou par voie topique intra-anale sur les muqueuses anales et intestinales, en l'absence de troubles de l'homéostasie du fer, ou ladite formulation topique A) étant administrée par voie nasale, vaginale ou anale en combinaison avec une formulation orale systémique B), contenant ledit mélange dans lequel la lactoferrine, le citrate de sodium et le bicarbonate de sodium sont présents dans la même plage de rapport molaire que ci-dessus, lorsque lesdites infections des muqueuses et le processus inflammatoire de celles-ci se manifestent avec une carence en fer, une anémie ou une anémie inflammatoire, ou ladite formulation orale systémique B) susmentionnée étant administrée en combinaison avec ladite formulation topique nasale A), lorsque la surcharge en fer cérébrale et les processus inflammatoires de celle-ci sont associés à la maladie d'Alzheimer.

**2.** La formulation topique A) et, si nécessaire, combinée à la formulation orale B) pour une utilisation selon la revendication 1, dans laquelle les effets suivants sont obtenus simultanément :

- ◦ inhibition significative de la multiplication des bactéries et virus pathogènes
- o inhibition de la formation de biofilm
- o inhibition de l'adhésivité bactérienne et virale
- ◦ inhibition de l'entrée des bactéries et virus pathogènes dans les cellules
- ◦ inhibition de la synthèse des cytokines pro-inflammatoires
- ◦ prévention et traitement de la MICI
- ◦ rétablissement de l'homéostasie du fer
- ◦ inhibition de la surcharge en fer intracérébrale
- ◦ rétablissement des valeurs physiologiques de l'hémoglobine, du nombre de globules rouges, du fer sérique total, de la ferritine sérique, du pourcentage d'hématocrite
- ◦ protection des lésions vaginales ou intestinales
- ◦ prévention et traitement de l'accouchement prématuré.

**3.** La formulation topique A) et, si nécessaire, combinée à la formulation orale B) pour une utilisation selon la revendication 1 ou 2, dans laquelle les infections aiguës ou chroniques sont choisies parmi des infections bactériennes causées par des bactéries sous forme planctonique ou dans des biofilms facultatifs ou obligatoires, d'adhésion ou intracellulaires.

4. La formulation topique A) et, si nécessaire, combinée à la formulation orale B) pour une utilisation selon l'une quelconque des revendications 1 à 3, où le traitement de la carence en fer, de l'anémie, ou de l'anémie inflammatoire avec ladite formulation orale systémique B) induit le rétablissement de la distribution physiologique du fer entre les tissus et les sécrétions et la circulation.

5. La formulation topique A) et, si nécessaire, combinée à la formulation orale B) pour une utilisation selon l'une quelconque des revendications 1 à 4, dans laquelle la lactoferrine dans la formulation topique A) et dans la formulation orale B) est en des quantités comprises entre 10 et 200 mg.

6. La formulation topique A) et, si nécessaire, combinée à la formulation orale B) pour une utilisation selon l'une quelconque des revendications 1 à 5, dans laquelle la lactoferrine a un degré de saturation en fer de 0 % à 100 %, de préférence de 0 % (limite incluse) à 100 % (limite non incluse), plus préférablement de 0 % à 20 % (limite incluse) de saturation.

7. La formulation topique A) et, si nécessaire, combinée à la formulation orale B) pour une utilisation selon l'une quelconque des revendications 1 à 6, dans laquelle le degré de saturation est obtenu avec un ou plusieurs des éléments Fe (III), Zn, Cu et Mn et dans laquelle la lactoferrine est choisie parmi une ou plusieurs lactoferrines intactes, le lobe N, le lobe C et les séquences contenant le site de liaison du fer.

8. La formulation topique A) et, si nécessaire, combinée à la formulation orale B), pour une utilisation selon l'une quelconque des revendications 1 à 7, dans laquelle ladite formulation topique nasale est sous forme de gouttes ou la formulation vaginale est sous forme de spray, de comprimés, d'ovules et de granulés, de préférence sous forme de comprimés ou d'ovules dissolubles, ou la formulation topique anale est sous forme de suppositoires ou de granulés dissolubles.

9. La formulation topique A) et, si nécessaire, combinée à la formulation orale B) pour une utilisation selon l'une quelconque des revendications 1 à 8, dans laquelle, par ladite administration, par voie intravaginale, intra-anale, de 10 à 200 mg de lactoferrine sont administrés deux fois ou plus par jour jusqu'à résolution de la pathologie.

10. La formulation topique A) et, si nécessaire, combinée à la formulation orale B) pour une utilisation selon l'une quelconque des revendications 1 à 7, étant administrée par la voie intranasale susmentionnée, plusieurs fois par jour dans des solutions contenant la lactoferrine à des concentrations comprises entre 0,1 et 10 % en poids/volume et la lactoferrine est de préférence contenue dans lesdites solutions en des quantités comprises entre 10 et 30 mg.

11. La formulation topique A) et, si nécessaire, combinée à la formulation orale B) pour une utilisation selon l'une quelconque des revendications 1 à 7, dans laquelle, lorsque lesdites formulations vaginales sont choisies parmi des comprimés ou des ovules ou les formulations anales solides sont choisies parmi des suppositoires ou des granulés solubles, elles contiennent entre 100 et 200 mg de lactoferrine et sont de préférence administrées au moins deux fois par jour.

12. La formulation topique A), si nécessaire, combinée à la formulation orale B) pour une utilisation selon l'une quelconque des revendications 1 à 10, dans laquelle la formulation orale contient une quantité totale de lactoferrine bovine de 100 mg à 200 mg à administrer deux fois ou plus par jour pendant au moins un mois.

13. Une composition orale comprenant comme principe actif un mélange de lactoferrine en des quantités supérieures à 200 mg jusqu'à 500 mg, de préférence entre 250 et 500 mg, et une teneur totale en citrate de sodium et bicarbonate de sodium comprise entre 140 et 240 mg, de préférence entre 170 et 200 mg, dans laquelle chacun desdits sels est présent en une quantité comprise entre 15 et 190 mg, de préférence entre 20 et 160 mg, pour une utilisation dans le traitement et la prévention d'infections virales par des virus à ADN, à ARN, enveloppés ou nus.

14. La composition orale pour une utilisation selon la revendication 13, dans laquelle le virus est le SARS-CoV-2 ou d'autres virus provoquant une surcharge en fer intracellulaire et extracellulaire et un orage inflammatoire.

# Fig.1

# Fig.2

BICARBONATE ION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007065482 A1
- EP 0454084 B1
- WO 2014097123 A1
- WO 2012084459 A1
- WO 2020194225 A1

### Non-patent literature cited in the description

- **BEDDEK, A.J** ; **SCHRYVERS, A.B**. The lactoferrin receptor complex in Gram negative bacteria. *Biometals*, 2010, vol. 23, 377-386
- **CALVANI F** ; **CUTONE A** ; **LEPANTO MS** ; **ROSA L** ; **VALENTINI V** ; **VALENTI P**. Efficacy of bovine lactoferrin in the post-surgical treatment of patients suffering from bisphosphonate-related osteonecrosis of the jaws: an open-label study. *Biometals*, June 2018, vol. 31 (3), 445-455
- **CAMPIONE E** ; **COSIO T** ; **ROSA L** ; **LANNA C** ; **DI GIROLAMO S** ; **GAZIANO R** ; **VALENTI P** ; **BIANCHI L**. Lactoferrin as Protective Natural Barrier of Respiratory and Intestinal Mucosa against Coronavirus Infection and Inflammation. *Int J Mol Sci.*, 11 July 2020, vol. 21 (14), 4903
- **MANCINELLI R** ; **ROSA L** ; **CUTONE A** ; **LEPANTO MS** ; **FRANCHITTO A** ; **ONORI P** ; **GAUDIO E** ; **VALENTI P**. Viral Hepatitis and Iron Dysregulation: Molecular Pathways and the Role of Lactoferrin. *Molecules*, 24 April 2020, vol. 25 (8), 1997
- **MOHAMED WA** ; **SCHAALAN MF**. Antidiabetic efficacy of lactoferrin in type 2 diabetic pediatrics; controlling impact on PPAR-γ, SIRT-1, and TLR4 downstream signaling pathway.. *DiabetolMetabSyndr.*, 04 December 2018, vol. 10, 89
- **PAESANO R** ; **NATALIZI T** ; **BERLUTTI F** ; **VALENTI P**. Body iron delocalization: the serious drawback in iron disorders in both developing and developed countries.. *Pathog Glob Health*, 2012
- **PETRIK, M** ; **ZHAI, C** ; **HAAS, H** ; **DECRISTOFORO, C.** Siderophores for molecular imaging applications. *Clin. Transl. Imaging*, 2017, vol. 5, 15-27
- **PUDDU, P.; LATORRE, D.; CAROLLO,M.; CATIZONE, A.; RICCI, G.; VALENTI, P.; GESSANI, S.** Bovine lactoferrin counteracts Toll-like receptor mediated activation signals in antigen presenting cells. *PLoS ONE*, 2011, vol. 6, e22504
- **ROSA L** ; **CUTONE A** ; **LEPANTO MS** ; **PAESANO R** ; **VALENTI P**. Lactoferrin: A Natural Glycoprotein Involved in Iron and Inflammatory Homeostasis. *Int J Mol Sci.*, 15 September 2017, vol. 18 (9)
- **ROSA L** ; **CUTONE A** ; **LEPANTO MS** ; **SCOTTI MJ** ; **CONTE MP** ; **PAESANO R** ; **VALENTI P**. Physicochemical properties influence the functions and efficacy of commercial bovine lactoferrins.. *Biometals.*, June 2018, vol. 31 (3), 301-312
- **VALENTI P** ; **ANTONINI G**. Lactoferrin: an important host defence against microbial and viral attack.. *Cell Mol Life Sci.*, November 2005, vol. 62 (22), 2576-87
- **ADLEROVA et al.** Lactoferrin a review. *Veterinarni Medicina*, 15 September 2008, vol. 53 (9), 457-468
- **STRATE VAN DER B W A**. *Antiviral activities of lactoferrin*, 01 December 2001, vol. 52 (3), 225-239XP
- **VALENTI P. et al.** Role of Lactobacilli and Lactoferrin in the Mucosal Cervicovaginal Defense. *Frontiers in Immunology*, 01 March 2018, vol. 9
- **LEPANTO M.S. et al.** Lactoferrin in Aseptic and Septic Inflammation. *Molecules*, 03 April 2019, vol. 24 (7), 1323
- **NONNECKE B J et al.** *Indian Journal Of Dairy Science*, 01 January 1984, vol. 67 (3), 606-613
- **JIANSHE LANG**. Inhibition of SARS Pseudovirus Cell Entry by Lactoferrin Binding to Heparan Sulfate. *PLoS ONE*, 01 August 2011, vol. 6 (8), e23710
- **MIRABELLI CARMEN**. Morphological Cell Profiling of SARS-CoV-2 Infection Identifies Drug Repurposing. *bioRxiv*, 10 June 2020, 1-36
- Comparative study of the iron-binding properties of human transferrins II. Electron paramagnetic resonance of mixed metal complexes of human lactotransferrin. **MAZURIER J et al.** Biochimica Et Biophysica Acta. Protein Structure And Molecular Enzymology. Elsevier, vol. 745